# EUROPEAN PATENT SPECIFICATION

(11) **EP 3 941 554 B1**
(45) Date of publication and mention of the grant of the patent: **11.06.2025**
(21) Application number: 20715758.7
(22) Date of filing: 17.03.2020
(51) Int. Cl.: A61M 11/00, A61M 11/04, A61M 15/06, A61M 16/00

(54) **AEROSOL DELIVERY SYSTEM**
AEROSOLABGABESYSTEM
SYSTÈME D'ADMINISTRATION D'AÉROSOL

(30) Priority: 21.03.2019 EP 19164461
(43) Date of publication of application: 26.01.2022
(73) Proprietor: Imperial Tobacco Limited, Bristol, BS3 2LL (GB)
(72) Inventor: LORD, Chris, Liverpool Merseyside L24 9HP (GB); SUDLOW, Thomas, Liverpool Merseyside L24 9HP (GB); ILLIDGE, Ben, Liverpool Merseyside L24 9HP (GB); MADDEN, Alfred, Liverpool Merseyside L24 9HP (GB); ASTBURY, Ben, Liverpool Merseyside L24 9HP (GB)
(74) Representative: Mewburn Ellis LLP
(86) International application number: PCT/EP2020/057331
(87) International publication number: WO 2020/187930

(56) References cited:
- WO-A1-2016/108174
- WO-A1-2018/197513
- WO-A2-2015/117705

## Description

### Field of the Invention

The present invention relates to an aerosol delivery system and an aerosol-generation apparatus for an aerosol delivery system. In particular, the present invention relates to an aerosol delivery system including a heater configured to heat an aerosol precursor to generate an aerosolised composition for inhalation by a user, and to an aerosol-generation apparatus therefor.

### Background

Pharmaceutical medicament, physiologically active substances and flavourings for example may be delivered to the human body by inhalation through the mouth and/or nose. Such material or substances may be delivered directly to the mucosa or mucous membrane lining the nasal and oral passages and/or the pulmonary system. For example, nicotine is consumed for therapeutic or recreational purposes and may be delivered to the body in a number of ways. Nicotine replacement therapies are aimed at people who wish to stop smoking and overcome their dependence on nicotine. Nicotine is delivered to the body in the form of aerosol delivery devices and systems, also known as smoking-substitute devices or nicotine delivery devices. Such devices may be non-powered or powered.

Devices or systems that are non-powered may comprise nicotine replacement therapy devices such as "inhalators", e.g. Nicorette^{®} Inhalator. These generally have the appearance of a plastic cigarette and are used by people who crave the behaviour associated with consumption of combustible tobacco - the so-called hand-to-mouth aspect - of smoking tobacco. Inhalators generally allow nicotine-containing aerosol to be inhaled through an elongate tube in which a container containing a nicotine carrier, for example, a substrate, is located. An air stream caused by suction through the tube by the user carries nicotine vapours into the lungs of the user to satisfy a nicotine craving. The container may comprise a replaceable cartridge, which includes a cartridge housing and a passageway in the housing in which a nicotine reservoir is located. The reservoir holds a measured amount of nicotine in the form of the nicotine carrier. The measured amount of nicotine is an amount suitable for delivering a specific number of "doses". The form of the nicotine carrier is such as to allow nicotine vapour to be released into a fluid stream passing around or through the reservoir. This process is known as aerosolization and or atomization. Aerosolization is the process or act of converting a physical substance into the form of particles small and light enough to be carried on the air i.e. into an aerosol. Atomization is the process or act of separating or reducing a physical substance into fine particles and may include the generation of aerosols. The passageway generally has an opening at each end for communication with the exterior of the housing and for allowing the fluid stream through the passageway. A nicotine-impermeable barrier seals the reservoir from atmosphere. The barrier includes passageway barrier portions for sealing the passageway on both sides of the reservoir. These barrier portions are frangible so as to be penetrable for opening the passageway to atmosphere.

A device or a system that is powered can fall into two sub-categories. In both subcategories, such devices or systems may comprise electronic devices or systems that permit a user to simulate the act of smoking by producing an aerosol mist or vapour that is drawn into the lungs through the mouth and then exhaled. The electronic devices or systems typically cause the vaporization of a liquid containing nicotine and entrainment of the vapour into an airstream. Vaporization of an element or compound is a phase transition from the liquid phase to vapour i.e. evaporation or boiling. In use, the user experiences a similar satisfaction and physical sensation to those experienced from a traditional smoking or tobacco product, and exhales an aerosol mist or vapour of similar appearance to the smoke exhaled when using such traditional smoking or tobacco products.

A person of ordinary skill in the art will appreciate that devices or systems of the second, powered category as used herein include, but are not limited to, electronic nicotine delivery systems, electronic cigarettes, e-cigarettes, e-cigs, vaping cigarettes, pipes, cigars, cigarillos, vaporizers and devices of a similar nature that function to produce an aerosol mist or vapour that is inhaled by a user. Such nicotine delivery devices or systems of the second category incorporate a liquid reservoir element generally including a vaporizer or misting element such as a heating element or other suitable element, and are known, *inter alia,* as atomizers, cartomizers, or clearomizers. Some electronic cigarettes are disposable; others are reusable, with replaceable and refillable parts.

Aerosol delivery devices or systems in a first sub-category of the second, powered category generally use heat and/or ultrasonic agitation to vaporize a solution comprising nicotine and/or other flavouring, propylene glycol and/or glycerine-based base into an aerosol mist of vapour for inhalation.

Aerosol delivery devices or systems in a second sub-category of the second, powered category may typically comprise devices or systems in which tobacco is heated rather than combusted. During use, volatile compounds may be released from the tobacco by heat transfer from the heat source and entrained in air drawn through the aerosol delivery device or system. Direct contact between a heat source of the aerosol delivery device or system and the tobacco heats the tobacco to form an aerosol. As the aerosol containing the released compounds passes through the device, it cools and condenses to form an aerosol for inhalation by the user. In such devices or systems, heating, as opposed to burning, the tobacco may reduce the odour that can arise through combustion and pyrolytic degradation of tobacco.

Aerosol delivery devices or systems falling into the first sub-category of powered devices or systems may typically comprise a powered unit, comprising a heater element, which is arranged to heat a portion of a carrier that holds an aerosol precursor. The carrier comprises a substrate formed of a "wicking" material, which can absorb aerosol precursor liquid from a reservoir and hold the aerosol precursor liquid. Upon activation of the heater element, aerosol precursor liquid in the portion of the carrier in the vicinity of the heater element is vaporised and released from the carrier into an airstream flowing around the heater and carrier. Released aerosol precursor is entrained into the airstream to be borne by the airstream to an outlet of the device or system, from where it can be inhaled by a user.

The heater element is typically a resistive coil heater, which is wrapped around a portion of the carrier and is usually located in the liquid reservoir of the device or system. Consequently, the surface of the heater may always be in contact with the aerosol precursor liquid, and long-term exposure may result in the degradation of either or both of the liquid and heater. Furthermore, residues may build up upon the surface of the heater element, which may result in undesirable toxicants being inhaled by the user. Furthermore, as the level of liquid in the reservoir diminishes through use, regions of the heater element may become exposed and overheat. WO2018197513 discloses an aerosol-generation apparatus comprising a heater having a planar heating surface, and is configured to receive an aerosol precursor carrier for thermal interaction the heating surface. The heater comprises a heating element at said heating surface and said heating surface comprises a fluid transport region adjacent said heating element. The fluid transport region is configured to move liquid across the heating surface towards the heating element.

The present technology has been devised in light of the above considerations.

### Summary of the Invention

An invention is set out in the claims. At its most general, the present technology proposes that an aerosol-generation apparatus has a heater and a fluid-transfer article for holding an aerosol precursor. A heating surface of the heater has at least one channel therein which opposes the fluid-transfer article. Normally, the fluid-transfer article will be arranged to transfer the aerosol precursor to an activation surface, and it is that activation surface of the fluid-transfer article which interacts with the heating surface. The channel may thus be open towards the activation surface.

Thus, the channel may define a spacing between part of the heating surface and the activation surface, through which spacing air can flow. It may thus form an air-flow pathway. Aerosol precursor which reaches the activation surface may then be heated by the heater, to form a vapour or a vapour/aerosol mixture. That vapour or mixture may then mix with air in the air-flow pathway to pass to the user.

Optionally, there may be a plurality of such channels, which plurality of channels forms the air-flow pathway.

Thus, according to a first aspect of the present disclosure, there may be provided an aerosol-generation apparatus comprising a heater and a fluid-transfer article, the fluid-transfer article having a first region for holding an aerosol precursor and for transferring said aerosol precursor to an activation surface of a second region of said article, said activation surface being configured for thermal interaction with a heating surface of said heater; said heating surface including at least one discontinuity therein forming a corresponding at least one channel, the or each said channel being configured for providing a fluid-flow pathway across said activation surface, said heater being configured such that, when the fluid transfer article is arranged with respect to said heating surface for thermal interaction therebetween, the or each said channel opposes said activation surface and opens towards said activation surface.

The activation surface may be disposed at an end of the fluid-transfer article.

Optionally, said heating surface is configured such that, when the fluid transfer article is arranged with respect to said heating surface for thermal interaction therebetween, the or each discontinuity is spaced apart from said activation surface.

Advantageously, the or each said channel may be at least partly defined by a pair of spaced apart side walls and an arcuate surface portion extending between said wall portions to form a ceiling portion of said channel.

Optionally, said arcuate surface portion blends smoothly with each of said side walls, thereby eliminating a sharp corner therebetween.

Alternatively, the or each channel may be at least partially defined by a pair of spaced apart side walls and a flat surface portion, said flat surface portion extending between said wall portions to form a ceiling portion of said channel. A further possibility is that the or each channel is at least partially defined by a pair of side walls, said side walls being inclined relative to each other to meet an apex portion of said channel.

Conveniently, said side walls are substantially planar.

Conveniently, at least said second region is formed from a polymeric wicking material.

Advantageously, said first and second regions are both formed from said polymeric wicking material.

Optionally, said polymeric wicking material is porous.

Conveniently, said polymeric wicking material is configured such that pore diameter in said first region is greater than pore diameter in said second region.

Advantageously, said polymeric wicking material is heat resistant.

Optionally, said polymeric wicking material is a hydrophilic material that is configured to transfer fluid from said first region to said second region.

Conveniently, said polymeric wicking material is of greater hydrophilicity in said second region than said first region.

According to another aspect of the present disclosure, there may be provided an aerosol delivery system having an aerosol-generation apparatus as discussed above and a carrier, the carrier having a housing containing said heater and said fluid-transfer article.

Preferably, said housing has an inlet and an outlet. The air-flow pathway may then extend to said inlet and said outlet, said air-flow pathway passing said arcuate surface portion of said heating surface.

The invention includes the combination of the aspects and preferred features described except where such a combination is clearly impermissible or expressly avoided.

### Summary of the Figures

So that the invention may be more readily understood, and so that further features thereof may be appreciated, embodiments of the invention will now be described by way of example with reference to the accompanying drawings in which:
**Figure 1****.** is a perspective view illustration of a system for aerosol delivery according to one or more embodiments of the present disclosure;
**Figure 2****.** is a cross-sectional side view illustration of part of an apparatus of the system for aerosol delivery of Figure 1;
**Figure 3****.** is a cross-sectional side view illustration of the system and apparatus for aerosol delivery of Figure 1;
**Figure 4****.** is a perspective view illustration of an aerosol carrier for use in the system for aerosol delivery according to one or more embodiments of the present disclosure;
**Figure 5****.** is a cross-section side view of elements of an aerosol carrier and of part of an apparatus of the system for aerosol delivery according to one or more embodiments of the present disclosure; invention
**Figure 6****.** is a cross-section side view of elements of an aerosol carrier and of part of an apparatus of the system for aerosol delivery according to one or more embodiments of the present disclosure;
**Figure 7****.** is a perspective view illustration of the aerosol carrier and of part of an apparatus of the system for aerosol delivery according to one or more embodiments of the present disclosure;
**Figure 8****.** is a perspective view illustration of the aerosol carrier and of part of an apparatus of the system for aerosol delivery according to one or more embodiments of the present invention;
**Figure 9****.** is a cross-section side view of an aerosol carrier according to one or more embodiments of the present disclosure;
**Figure 10****.** is a perspective cross-section side view of the aerosol carrier of Figure 11; and
Figure 11. is an exploded perspective view illustration of a kit-of-parts for assembling a system according to one or more embodiments of the present disclosure;

### Detailed Description of the technology

Aspects and embodiments of the present disclosure will now be discussed with reference to the accompanying figures. Further aspects and embodiments will be apparent to those skilled in the art.

In general outline, one or more embodiments in accordance with the present disclosure may provide a system for aerosol delivery in which an aerosol carrier may be inserted into a receptacle (e.g. a "heating chamber") of an apparatus for initiating and maintaining release of an aerosol from the aerosol carrier. Another end, or another end portion, of the aerosol carrier may protrude from the apparatus and can be inserted into the mouth of a user for the inhalation of aerosol released from the aerosol carrier cartridge during operation of the apparatus.

Hereinafter, and for convenience only, "system for aerosol delivery" shall be referred to as "aerosol delivery system".

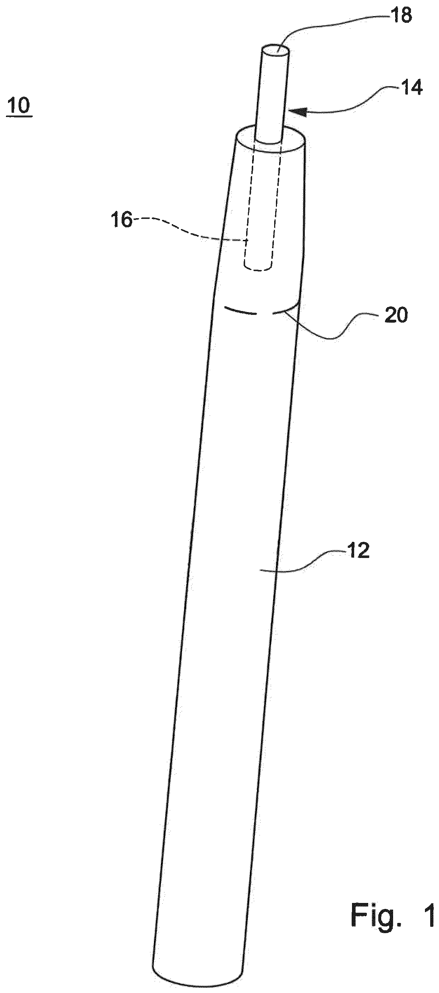

Referring now to Figure 1, there is illustrated a perspective view of an aerosol delivery system 10 comprising an aerosol generation apparatus 12 operative to initiate and maintain release of aerosol from a fluid-transfer article in an aerosol carrier 14. In the arrangement of Figure 1, the aerosol carrier 14 is shown with a first end 16 thereof and a portion of the length of the aerosol carrier 14 located within a receptacle of the apparatus 12. A remaining portion of the aerosol carrier 14 extends out of the receptacle. This remaining portion of the aerosol carrier 14, terminating at a second end 18 of the aerosol carrier, is configured for insertion into a user's mouth. A vapour and/or aerosol is produced when a heater (not shown in Figure 1) of the apparatus 12 heats a fluid-transfer article in the aerosol carrier 14 to release a vapour and/or an aerosol, and this can be delivered to the user, when the user sucks or inhales, via a fluid passage in communication with an outlet of the aerosol carrier 14 from the fluid-transfer article to the second end 18.

The device 12 also comprises air-intake apertures 20 in the housing of the apparatus 12 to provide a passage for air to be drawn into the interior of the apparatus 12 (when the user sucks or inhales) for delivery to the first end 16 of the aerosol carrier 14, so that the air can be drawn across an activation surface of a fluid-transfer article located within a housing of the aerosol carrier cartridge 14 during use. Optionally, these apertures may be perforations in the housing of the apparatus 12.

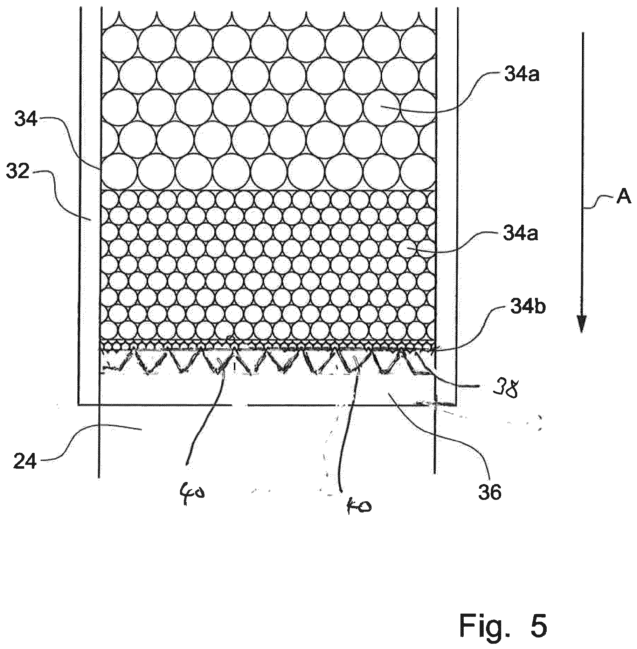

A fluid-transfer article (not shown in Figure 1, but described hereinafter with reference to Figs. 5, 6, 7, 8, 9, 10, 11 and 12) is located within a housing of the aerosol carrier 14. The fluid-transfer article contains an aerosol precursor material, which may include at least one of: nicotine; a nicotine precursor material; a nicotine compound; and one or more flavourings. The fluid-transfer article is located within the housing of the aerosol carrier 14 to allow air drawn into the aerosol carrier 14 at, or proximal, the first end 16 to flow across an activation surface of the fluid-transfer article. As air passes across the activation surface of the fluid-transfer article, an aerosol may be entrained in the air stream from a substrate forming the fluid-transfer article, e.g. via diffusion from the substrate to the air stream and/or via vaporisation of the aerosol precursor material and release from the fluid-transfer article under heating.

The substrate forming the fluid-transfer article 34 comprises a porous material where pores of the porous material hold, contain, carry, or bear the aerosol precursor material. In particular, the porous material of the fluid-transfer article may be a polymeric wicking material such as, for example, a sintered material. Particular examples of material suitable for the fluid-transfer article include: Polyetherimide (PEI); Polytetrafluoroethylene (PTFE); Polyether ether ketone (PEEK); Polyimide (PI); Polyethersulphone (PES); and Ultra-High Molecular Weight Polyethylene. Other suitable materials may comprise, for example, BioVyonTM (by Porvair Filtration Group Ltd) and materials available from Porex^{®}. Further optionally, a substrate forming the fluid-transfer article may comprise Polypropylene (PP) or Polyethylene Terephthalate (PET). All such materials may be described as heat resistant polymeric wicking material in the context of the present technology.

The aerosol carrier 14 is removable from the apparatus 12 so that it may be disposed of when expired. After removal of a used aerosol carrier 14, a replacement aerosol carrier 14 can be inserted into the apparatus 12 to replace the used aerosol carrier 14.

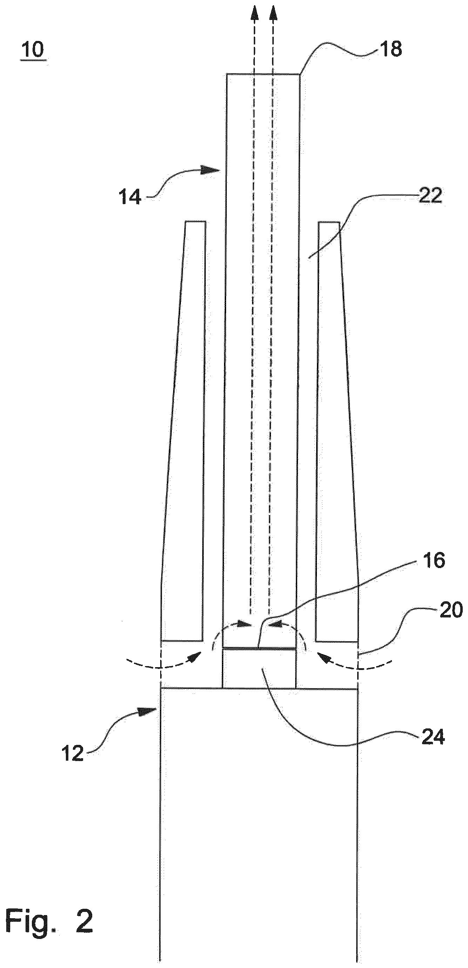

Figure 2 is a cross-sectional side view illustration of a part of apparatus 12 of the aerosol delivery system 10. The apparatus 12 comprises a receptacle 22 in which is located a portion of the aerosol carrier 14. In one or more optional arrangements, the receptacle 22 may enclose the aerosol carrier 14. The apparatus 12 also comprise a heater 24, which opposes an activation surface of the fluid-transfer article (not shown in Figure 2) of the aerosol carrier 14 when an aerosol carrier 14 is located within the receptacle 22.

Air flows into the apparatus 12 (in particular, into a closed end of the receptacle 22) via air-intake apertures 20. From the closed end of the receptacle 22, the air is drawn into the aerosol carrier 14 (under the action of the user inhaling or sucking on the second end 18) and expelled at the second end 18. As the air flows into the aerosol carrier 14, it passes across the activation surface of the fluid-transfer article. Heat from the heater 24, which opposes the activation surface of the fluid-transfer article, causes vaporisation of aerosol precursor material at the activation surface of the fluid-transfer article and an aerosol is created in the air flowing over the activation surface. Thus, through the application of heat in the region of the activation surface of the fluid-transfer article, an aerosol is released, or liberated, from the fluid-transfer article, and is drawn from the material of the aerosol carrier unit by the air flowing across the activation surface and is transported in the air flow to via outlet conduits (not shown in Figure 2) in the housing of the aerosol carrier 14 to the second end 18. The direction of air flow is illustrated by arrows in Figure 2.

To achieve release of the captive aerosol from the fluid-transfer article, the fluid-transfer article of the aerosol carrier 14 is heated by the heater 24. As a user sucks or inhales on second end 18 of the aerosol carrier 14, the aerosol released from the fluid-transfer article and entrained in the air flowing across the activation surface of the fluid-transfer article is drawn through the outlet conduits (not shown) in the housing of the aerosol carrier 14 towards the second end 18 and onwards into the user's mouth.

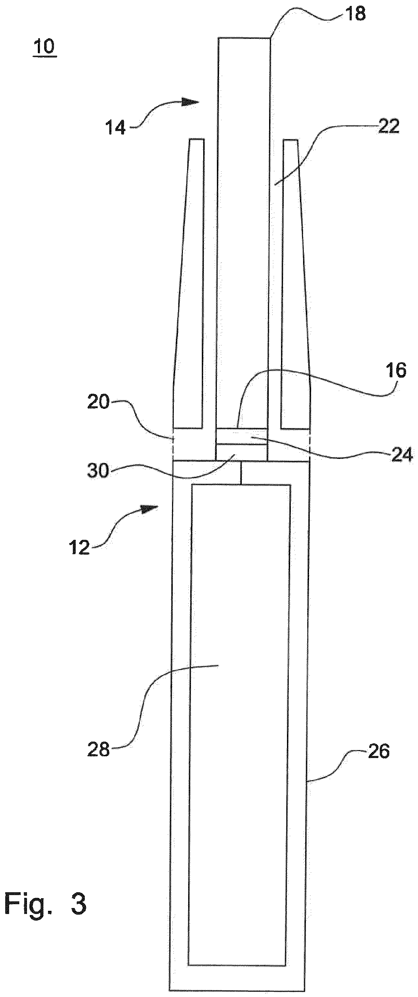

Turning now to Figure 3, a cross-sectional side view of the aerosol delivery system 10 is schematically illustrated showing the features described above in relation to Figures 1 and 2 in more detail. As can be seen, apparatus 12 comprises a housing 26, in which are located the receptacle 22 and heater 24. The housing 26 also contains control circuitry (not shown) operative by a user, or upon detection of air and/or vapour being drawn into the device 12 through air-intake apertures 20, i.e. when the user sucks or inhales. Additionally, the housing 26 comprises an electrical energy supply 28, for example a battery. Optionally, the battery comprises a rechargeable lithium ion battery. The housing 26 also comprises a coupling 30 for electrically (and optionally mechanically) coupling the electrical energy supply 28 to control circuitry (not shown) for powering and controlling operation of the heater 24.

Responsive to activation of the control circuitry of apparatus 12, the heater 24 heats the fluid-transfer article (not shown in Figure 3) of aerosol carrier 14. This heating process initiates (and, through continued operation, maintains) release of vapour and/or an aerosol from the activation surface of the fluid-transfer article. The vapour and/or aerosol formed as a result of the heating process is entrained into a stream of air being drawn across the activation surface of the fluid-transfer article (as the user sucks or inhales). The stream of air with the entrained vapour and/or aerosol passes through the aerosol carrier 14 via outlet conduits (not shown) and exits the aerosol carrier 14 at second end 18 for delivery to the user. This process is briefly described above in relation to Figure 2, where arrows schematically denote the flow of the air stream into the device 12 and through the aerosol carrier 14, and the flow of the air stream with the entrained vapour and/or aerosol through the aerosol carrier cartridge 14.

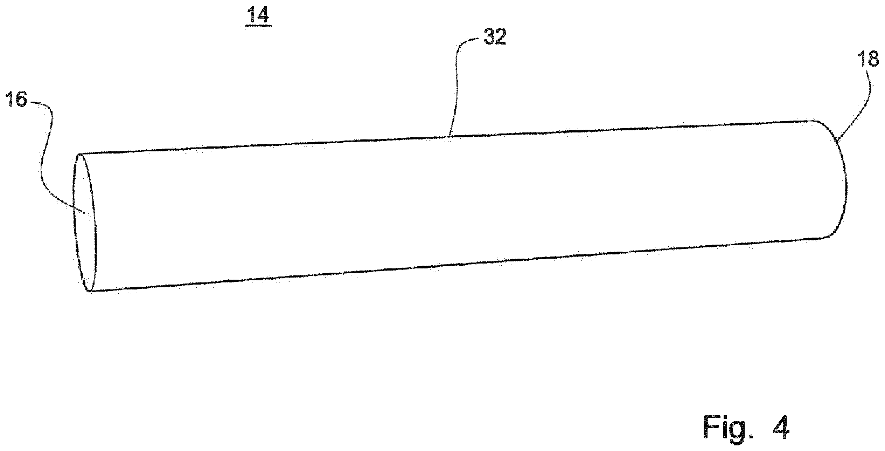

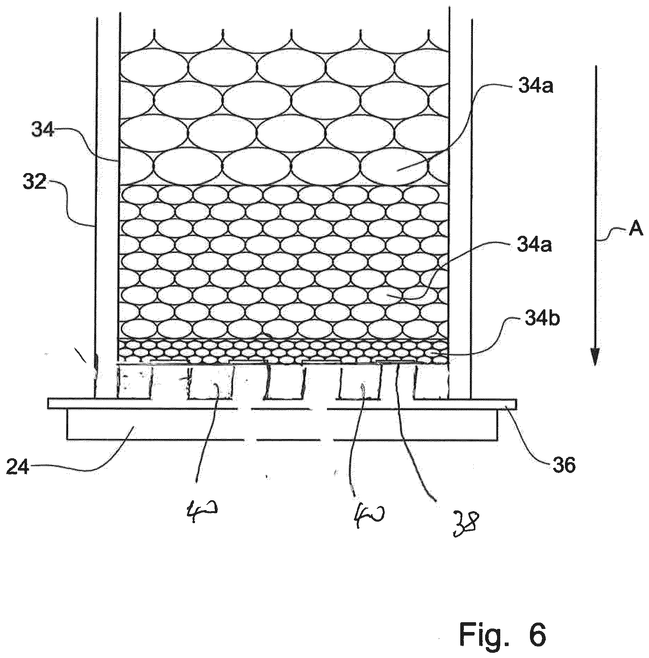

Figures 4 to 6 schematically illustrate the aerosol carrier 14 in more detail (and, in Figures 5 and 6, features within the receptacle in more detail). Figure 4 illustrates an exterior of the aerosol carrier 14, Figure 5 illustrates internal components of the aerosol carrier 14 in an optional arrangement, and Fig. 6 illustrates internal components of the aerosol carrier 14 in another optional arrangement.

Fig. 4 illustrates the exterior of the aerosol carrier 14, which comprises housing 32 for housing said fluid-transfer article (not shown) and at least one other internal component. The particular housing 32 illustrated in Figure 4 comprises a tubular member, which may be generally cylindrical in form, and which is configured to be received within the receptacle of the apparatus. First end 16 of the aerosol carrier 14 is for location to oppose the heater of the apparatus, and second end 18 (and the region adjacent the second end 18) is configured for insertion into a user's mouth.

Figure 5 illustrates some internal components of the aerosol carrier 14 and of the heater 24 of apparatus 12.

As described above, the aerosol carrier 14 comprises a fluid-transfer article 34. The aerosol carrier 14 optionally may comprise a conduction element 36 (as shown in Figure 5) being part of the heater 24. In one or more arrangements, the aerosol carrier 14 is located within the receptacle of the apparatus such that the activation surface of the fluid-transfer article opposes the heater of the apparatus and receives heat directly from the heater of the apparatus. In an optional arrangement, such as illustrated in Figure 5 for example, the aerosol carrier 14 comprises a conduction element 36. When aerosol carrier 14 is located within the receptacle of the apparatus such that an activation surface 38 of the fluid-transfer article 34 is located to oppose the heater 24 of the apparatus, the conduction element 36 is disposed between the rest of the heater 24 and the activation surface 38 of the fluid-transfer article 34. Heat may be transferred to the activation surface via conduction through conduction element 36 (i.e. application of heat to the activation surface is indirect).

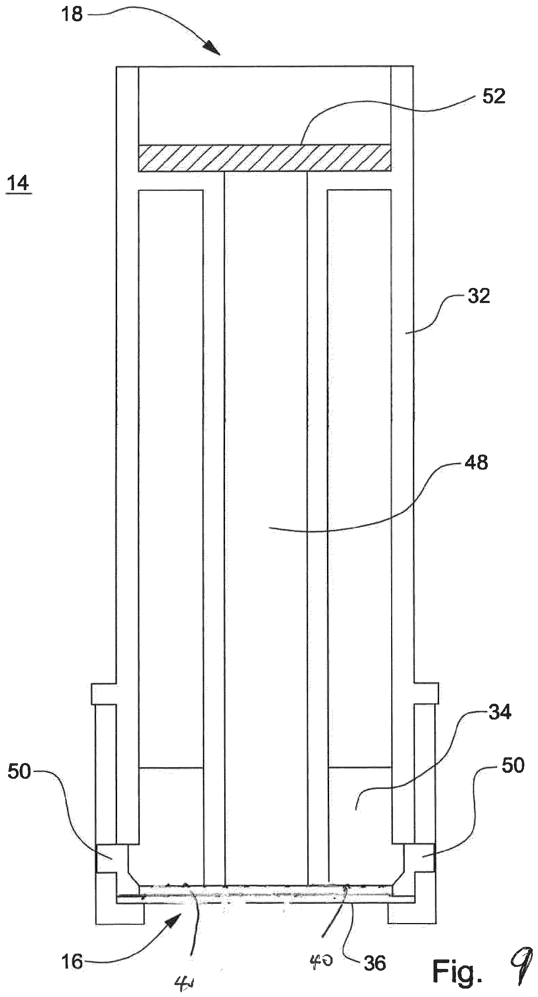

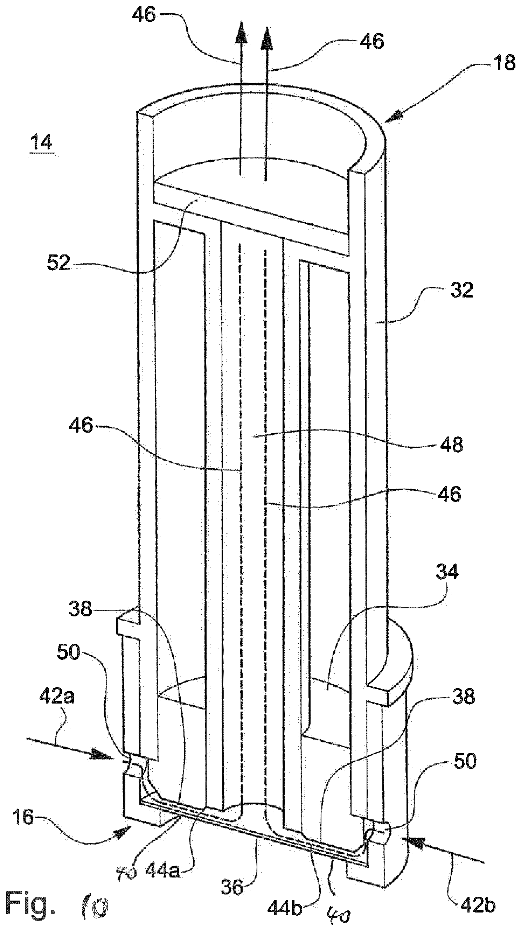

Further components not shown in Figures. 5 and 6 (see Figures 9 and 10) comprise: an inlet conduit, via which air can be drawn into the aerosol carrier 14; an outlet conduit, via which an air stream entrained with aerosol can be drawn from the aerosol carrier 14; a filter element; and a reservoir for storing aerosol precursor material and for providing the aerosol precursor material to the fluid-transfer article 34.

In Figures 5 and 6, aerosol carrier is shown as comprising the fluid-transfer article 34 located within housing 32. The material forming the fluid transfer article 34 comprises a porous structure, where pore diameter size varies between one end of the fluid-transfer article 34 and another end of the fluid-transfer article. In the illustrative examples of Figs. 5 and 6, the pore diameter size gradually decreases from a first end remote from heater 24 (the upper end as shown in the figure) to a second end proximal heater 24 (the lower end as shown in the figure). Although the figure illustrates the pore diameter size changing in a step-wise manner from the first to the second end (i.e. a first region with pores having a diameter of a first size, a second region with pores having a diameter of a second, smaller size, and a third region with pores having a diameter of a third, yet smaller size), the change in pore size from the first end to the second end may be gradual rather than step-wise. This configuration of pores having a decreasing diameter size from the first end and second end can provide a wicking effect, which can serve to draw fluid from the first end to the second end of the fluid-transfer article 34.

The fluid-transfer article 34 comprises a first region 34a for holding an aerosol precursor. In one or more arrangements, the first region 34a of the fluid-transfer article 34 comprises a reservoir for holding the aerosol precursor. The first region 34a can be the sole reservoir of the aerosol carrier 14, or it can be arranged in fluid communication with a separate reservoir, where aerosol precursor is stored for supply to the first region 34a.

The fluid-transfer article 34 also comprises a second region 34b. Aerosol precursor is drawn from the first region 34a to the second region 34b by the wicking effect of the substrate material forming the fluid transfer article. Thus, the first region 34a is configured to transfer the aerosol precursor to the second region 34b of the article 34.

At the second end of fluid-transfer article 34, the surface of the second region 34b defines the activation surface 38, which is disposed opposite a surface for conveying heat to the activation surface 38. In the illustrative examples of Figures. 5 and 6, the opposing surface for conveying heat to the activation surface 38 comprises a conduction element 36 which is a part of the heater 24. The conduction element 36 is located for thermal interaction with the rest of the heater 24 and is arranged to transfer heat from the rest of the heater 24 to the activation surface 38. As noted above, however, the conduction element 36 may be absent in some arrangements, in which case the activation surface 38 is disposed to receive heat directly from heater 24.

The conduction element 36, if present, may comprise a thin film of thermally conductive material, such as, for example, a metal foil (for example, aluminium, brass, copper, gold, steel, silver, or an alloy comprising anyone of the foregoing together with thermally conductive plastics and/or ceramics).

The surface of the conduction element 36 is discontinuous such that at least one channel 40 is formed between the activation surface 38 and the conduction element 36 (or the upper surface of the heater 24 is discontinuous in the case of arrangements in which the conduction element 36 is absent). In some arrangements, the discontinuities may be such that the surface of the conduction element 36 or heater 24 itself is undulating.

In the illustrative examples of Figures 5 and 6, the conduction element 36 has a plurality of grooves or valleys therein to form an undulating surface, the grooves or valleys being disposed in a parallel arrangement in the conduction element 36. Since it is the surface of the conduction element 36 closest to the activation surface 38 which acts as the heating surface for the aerosol precursor, those grooves or valleys can be said to be in the heating surface. The grooves or valleys define a plurality of channels 40, between the activation surface 38 and the conduction element 36.

In the illustrative example of Figure 5, the grooves or valleys in the conduction element 36 provide alternating peaks and troughs that give rise to a "saw-tooth" type profile. In one or more optional arrangements, the surface of the conduction element 36 may comprise a "castellated" type profile (i.e. a "square wave" type profile), for example, such as illustrated in the example of Figure. 6. In one or more optional arrangements, the surface of the conduction element 36 may comprise a "sinusoidal" type profile. The profile may comprise a mixture of two or more of the above profiles given as illustrative examples.

In the illustrative examples of Figures 5 and 6, the first region 34a of the fluid-transfer article 34 is located at an "upstream" end of the fluid-transfer article 34 and the second region 34b is located at a downstream" end of the fluid-transfer article 34. That is, aerosol precursor is wicked, or is drawn, from the "upstream" end of the fluid-transfer article 34 to the "downstream" end of the fluid-transfer article 34 (as denoted by arrow A in Figure 5).

The aerosol precursor is configured to release an aerosol and/or vapour upon heating. Thus, when the activation surface 38 receives heat conveyed from heater 24, the aerosol precursor held at the activation surface 38 is heated. The aerosol precursor, which is captively held in material of the fluid-transfer article at the activation surface 38 is released into an air stream flowing through the channels 40 between the conduction element 36 and activation surface 38 (or between the heater 24 and the activation surface 38) as an aerosol and/or vapour.

The shape and/or configuration of the conduction element 36 (or the upper surface of the heater 24 if no conduction element is present) and the associated shape(s) and/or configuration(s) of the one or more channels 40 formed between the activation surface 38 and conduction element 36 (or between the activation surface 38 and heater 24) permit air to flow across the activation surface 38 (through the one or more channels 40) and also increase the surface area of the activation surface 38 of the fluid-transfer article 34 that is available for contact with a flow of air across the activation surface 38.

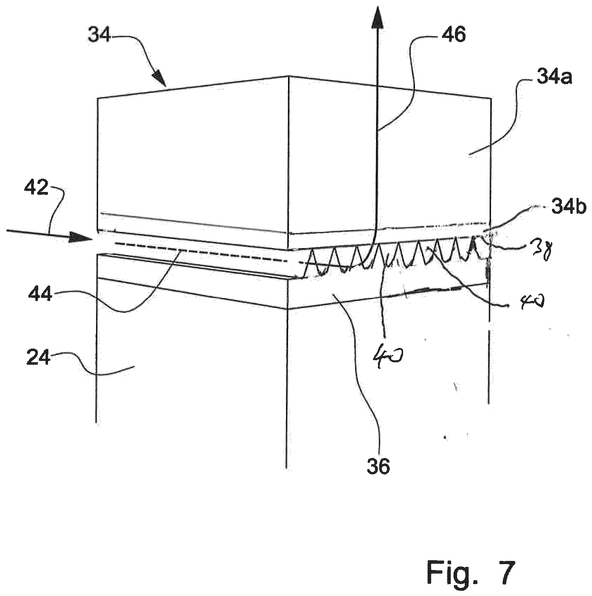

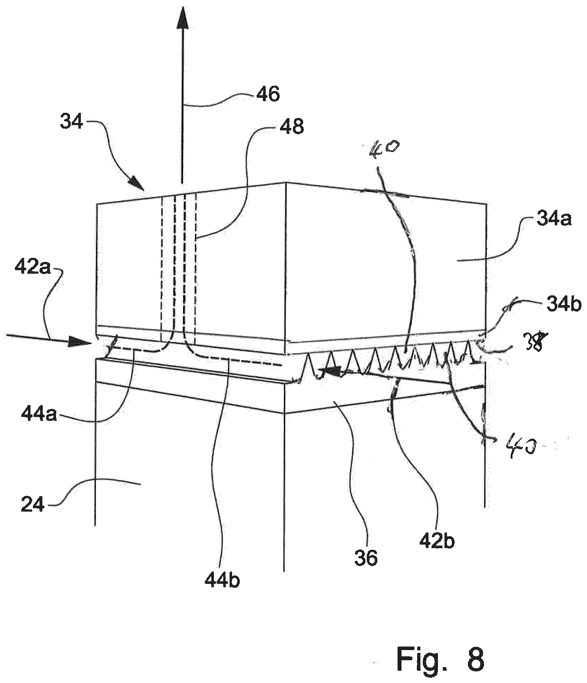

Figures 7 and 8 show perspective view illustrations of the fluid-transfer article 34 of the aerosol carrier and a heater 24 of the apparatus of the system for aerosol delivery. In particular, these figures illustrate air flows across the activation surface 38 when the apparatus is in use in a first arrangement of the fluid-transfer article 34 (see Figure 7), and in a second arrangement of the fluid-transfer article 34 (see Figure 8).

In the illustrated example of use of the apparatus schematically illustrated in Figure 7, when a user sucks on a mouthpiece of the apparatus, air is drawn into the carrier through inlet apertures (not shown) provided in a housing of the carrier. An incoming air stream 42 is directed to the activation surface 38 of the fluid-transfer article 34 (e.g. via a fluid communication pathway within the housing of the carrier). When the incoming air stream 42 reaches a first side of the activation surface 38, the incoming air stream 42 flows across the activation surface 38 via the one or more channels 40 formed between the activation surface 38 and the conduction element 36 (or between the activation surface 38 and heater 24). The air stream flowing through the one or more channels 40 is denoted by dashed line 44 in Figure 7. As the air stream 44 flows through the one or more channels 40, aerosol precursor at activation surface 38, across which the air stream 44 flows, is released from the activation surface 38 by heat conveyed to the activation surface from the heater 24. Aerosol precursor released from the activation surface 38 in this manner is then entrained in the air stream 44 flowing through the one or more channels 40.

In use, the heater 24 of the apparatus 12 conveys heat to the fluid transfer article 34 to raise the temperature of the activation surface 38 to a sufficient temperature to release, or liberate, captive substances (i.e. the aerosol precursor) held at the activation surface 38 of the fluid-transfer article 34 to form a vapour and/or aerosol, which is drawn downstream across the activation surface 38 of the fluid-transfer article. As the air stream 44 continues its passage in the one or more channels 40, more released aerosol precursor is entrained within the air stream 44. When the air stream 44 entrained with aerosol precursor exits the one or more channels 40 at a second side of the activation surface 38, it is directed to an outlet, from where it can be inhaled by the user via a mouthpiece. An outgoing air stream 46 entrained with aerosol precursor is directed to the outlet (e.g. via a fluid communication pathway within the housing of the carrier).

Therefore, operation of the apparatus will cause heat from the heater 24 to be conveyed to the activation surface 38 of the fluid-transfer article. At a sufficiently high temperature, captive substances held at the activation surface 38 of the fluid-transfer article 34 are released, or liberated, to form a vapour and/or aerosol. Thus, when a user draws on a mouthpiece of the apparatus, the released substances from the fluid-transfer article are drawn away from the activation surface 38 (entrained in a stream of air) and condense to form an aerosol that is drawn through the a gas communication pathway for delivery to an outlet, which is in fluid communication with the mouthpiece.

As the aerosol precursor is released from the activation surface 38, a wicking effect of the fluid-transfer article 34 causes aerosol precursor within the body of the fluid-transfer article to migrate to the activation surface 38 to replace the aerosol precursor released from the activation surface 38 into air stream 44.

Operation of the heater 24 is controlled by control circuitry (not shown), which is operable to actuate the heater 24 responsive to an actuation signal from a switch operable by a user or configured to detect when the user draws air through a mouthpiece of the apparatus by sucking or inhaling. In an optional arrangement, the control circuitry operates to actuate the heater 24 with as little delay as possible from receipt of the actuation signal from the switch, or detection of the user drawing air through the mouthpiece. This may effect near instantaneous heating of the activation surface 38 of the fluid-transfer article 34.

In the illustrated example of use of the apparatus schematically illustrated in Figure 8, rather than the case of Figure 7 where air is drawn toward the activation surface 38 from one side only (and exits from the one or more channels 40 at an opposite side), a gas communication pathway for an incoming air stream is configured to deliver the incoming air stream to the activation surface 38 from both sides of the fluid-transfer article, and thus from both ends of the channels 40 formed therein. In such an arrangement, a gas communication pathway for an outlet airstream may be provided through the body of the fluid-transfer article 34. An outlet fluid communication pathway for an outlet airstream in the illustrative example of Figure 8 is denoted by reference number 48.

Thus, in the illustrative example of Figure 8, when a user draws on a mouthpiece of the apparatus, air is drawn into the carrier 14 through inlet apertures (not shown) provided in a housing of the carrier. An incoming air stream 42a from a first side is directed to a first side of the activation surface 38 of the fluid-transfer article 34 (e.g. via a gas communication pathway within the housing of the carrier 14). An incoming air stream 42b from a second side is directed to a second side of the activation surface 38 of the fluid-transfer article 34 (e.g. via a gas communication pathway within the housing of the carrier 14). When the incoming air stream 42a from the first side reaches the first side of the activation surface 38, the incoming air stream 42a flows across the activation surface 38 via the one or more channels 40 formed between the activation surface 38 and the conduction element 36 (or between the activation surface 38 and heater 24). Likewise, when the incoming air stream 42b from the second side reaches the second side of the activation surface 38, the incoming air stream 42b flows across the activation surface 38 via the one or more channels 40 formed between the activation surface 38 and the conduction element 36 (or between the activation surface 38 and heater 24). The air streams 42a, 42b from each side flowing through the one or more channels 40 are denoted by dashed lines 44a and 44b in Figure 8.

As air streams 44a and 44b flow through the one or more channels 40, aerosol precursor in the activation surface 38, across which the air streams 44a and 44b flow, is released from the activation surface 38 by heat conveyed to the activation surface from the heater 24. Aerosol precursor released from the activation surface 38 is entrained in air streams 44a and 44b flowing through the one or more channels 40. In use, the heater 24 of the apparatus 12 conveys heat to the fluid-transfer article 34 to raise a temperature of the activation surface 38 to a sufficient temperature to release, or liberate, captive substances (i.e. the aerosol precursor) held at the activation surface 38 of the fluid-transfer article 34 to form a vapour and/or aerosol, which is drawn downstream across the activation surface 38 of the fluid-transfer article.

As the air streams 44a and 44b continue their passages in the one or more channels 40, more released aerosol precursor is entrained within the air streams 44a and 44b. When the air streams 44a and 44b entrained with aerosol precursor meet at a mouth of the outlet fluid communication pathway 48, they enter the outlet fluid communication pathway 48 and continue until they exit outlet fluid communication pathway 48, either as a single outgoing air stream 46 (as shown), or as separate outgoing air streams. The outgoing air stream 46 is directed to an outlet, from where it can be inhaled by the user via a mouthpiece. The outgoing air stream 46 entrained with aerosol precursor is directed to the outlet (e.g. via a gas communication pathway within the housing of the carrier 14).

Figures 9 and 10 illustrate an aerosol carrier 14 according to one or more possible arrangements in more detail. Figure 9 is a cross-section side view illustration of the aerosol carrier 14 and Figure 10 is a perspective cross-section side view illustration of the aerosol carrier 14 of Figure 9.

As can be seen from Figures 9 and 10, the aerosol carrier 14 is generally tubular in form. The aerosol carrier 14 comprises housing 32, which defines the external walls of the aerosol carrier 14 and which defines therein a chamber in which are disposed the fluid-transfer article 34 (adjacent the first end 16 of the aerosol carrier 14) and internal walls defining the fluid communication pathway 48. Fluid communication pathway 48 defines a fluid pathway for an outgoing air stream from the channels 40 to the second end 18 of the aerosol carrier 14. In the examples illustrated in Figures 9 and 10, the fluid-transfer article 34 is an annular shaped element located around the fluid communication pathway 48. Note that, in Figures 9 and 10, the channels 40 in the conduction element 36 extend radially and the sectional views of Figures 9 and 10 are along the length of two channels on opposite radial positions relative to the fluid communication pathway 48 in the fluid-transfer article..

In walls of the housing 32, there are provided inlet apertures 50 to provide a fluid communication pathway for an incoming air stream to reach the fluid-transfer article 34, and particularly the one or more channels 40 defined between the activation surface of the fluid-transfer article 34 and the conduction element 36 (or between the activation surface and the 15 heater).

In the illustrated example of Figures 9 and 10, the aerosol carrier 14 further comprises a filter element 52. The filter element 52 is located across the fluid communication pathway 48 such that an outgoing air stream passing through the fluid communication pathway 48 passes through the filter element 52.

With reference to Figure 10, when a user sucks on a mouthpiece of the apparatus (or on the second end 18 of the aerosol carrier 14, if configured as a mouthpiece), air is drawn into the carrier through inlet apertures 50 extending through walls in the housing 32 of the aerosol carrier 14. An incoming air stream 42a from a first side of the aerosol carrier 14 is directed to a first side of the activation surface 38 of the fluid-transfer article 34 (e.g. via a gas communication pathway within the housing of the carrier). An incoming air stream 42b from a second side of the aerosol carrier 14 is directed to a second side of the activation surface 38 of the fluid-transfer article 34 (e.g. via a gas communication pathway within the housing of the carrier). When the incoming air stream 42a from the first side of the aerosol carrier 14 reaches the first side of the activation surface 38, the incoming air stream 42a from the first side of the aerosol carrier 14 flows across the activation surface 38 via the one or more channels 40 formed between the activation surface 38 and the conduction element 36 (or between the activation surface 38 and heater 24). Likewise, when the incoming air stream 42b from the second side of the aerosol carrier 14 reaches the second side of the activation surface 38, the incoming air stream 42b from the second side of the aerosol carrier 14 flows across the activation surface 38 via the one or more channels 40 formed between the activation surface 38 and the conduction element 36 (or between the activation surface 38 and heater 24). The air streams from each side flowing through the one or more channels 40 are denoted by dashed lines 44a and 44b in Figure 12. As air streams 44a and 44b flow through the one or more channels 40, aerosol precursor in the activation surface 38, across which the air streams 44a and 44b flow, is released from the activation surface 38 by heat conveyed to the activation surface from the heater 24. Aerosol precursor released from the activation surface 38 is entrained in air streams 44a and 44b flowing through the one or more channels 40.

In use, the heater 24 of the apparatus 12 conveys heat to the activation surface 38 of the fluid-transfer article 34 to raise a temperature of the activation surface 38 to a sufficient temperature to release, or liberate, captive substances (i.e. the aerosol precursor) held at the activation surface 38 of the fluid-transfer article 34 to form a vapour and/or aerosol, which is drawn downstream across the activation surface 38 of the fluid-transfer article 34.

As the air streams 44a and 44b continue their passages in the one or more channels 40, more released aerosol precursor is entrained within the air streams 44a and 44b. When the air streams 44a and 44b entrained with aerosol precursor meet at a mouth of the outlet fluid communication pathway 48, they enter the outlet fluid communication pathway 48 and continue until they pass through filter element 52 and exit outlet fluid communication pathway 48, either as a single outgoing air stream, or as separate outgoing air streams 46 (as shown). The outgoing air streams 46 are directed to an outlet, from where it can be inhaled by the user directly (if the second end 18 of the aerosol capsule 14 is configured as a mouthpiece), or via a mouthpiece. The outgoing air streams 46 entrained with aerosol precursor are directed to the outlet (e.g. via a gas communication pathway within the housing of the carrier).

When the user initially draws on a mouthpiece of the apparatus (or one the second end 18 of the aerosol carrier 14, if configured as a mouthpiece), this will cause an air column located in the fluid communication pathway 48 to move towards the outlet. In tum, this will draw air into the fluid communication pathway from the one or more channels 40. This will cause a pressure drop in the channels 40. To equalise the pressure in the channels 40, air will be drawn into the aerosol carrier 14, and thus into the channels 40 via the inlet apertures 50. During the period of lower pressure in the one or more channels 40 when the user begins to draw, aerosol precursor in the fluid-transfer medium will be released into the channels from the activation surface 38, because the aerosol precursor is drawn into the one or more channels by way of the lower pressure. This effect is in addition to the effect of releasing the aerosol precursor from the activation surface 38 by way of heat conveyed from the heater. The drawing of the aerosol precursor from the activation surface 38 by way of the user sucking on the mouthpiece of the apparatus (or one the second end 18 of the aerosol carrier 14, if configured as a mouthpiece) may produce a dragging effect on the volumetric rate of flow experienced by the user during a suction action, i.e. the user may have to suck harder to achieve a same volumetric rate of flow. This effect may manifest itself as a similar physical sensation experienced by the user as those experienced from a traditional smoking or tobacco product.

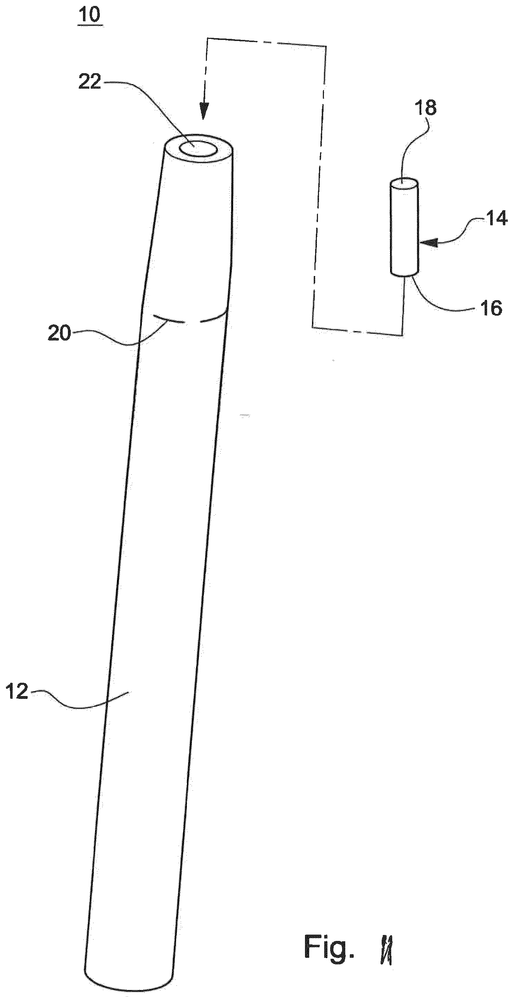

Figure 11 is an exploded perspective view illustration of a kit-of-parts for assembling an aerosol delivery system 10.

As will be appreciated, in the arrangements described above, the fluid-transfer article 34 is provided within a housing 32 of the aerosol carrier 14. In such arrangements, the housing of the carrier 14 serves to protect the aerosol precursor-containing fluid-transfer article 34, whilst also allowing the carrier 14 to be handled by a user without his/her fingers coming into contact with the aerosol precursor liquid retained therein. In such arrangements, it will be appreciated that the carrier 14 has a multi-part construction. The second region 34b of the fluid-transfer article may have a thickness of less than 5mm. In other embodiments it may have a thickness of: less than 3.5mm, less than 3mm, less than 2.5mm, less than 2mm, less than 1.9mm, less than 1.8mm, less than 1.7mm, less than 1.6mm, less than 1.5mm, less than 1.4mm, less than 1.3mm, less than 1.2mm, less than 1.1mm, less than 1mm, less than 0.9mm, less than 0.8mm, less than 0.7mm, less than 0.6mm, less than 0.5mm, less than 0.4mm, less than 0.3mm, less than 0.2mm, or less than 0.1mm.

There has been described in the foregoing one or more proposals for an aerosol delivery system, and parts thereof, that avoids or at least ameliorates problems of the prior art.

In one or more optional arrangements, a fluid-transfer article 34 containing nicotine and/or nicotine compounds may be substituted or supplemented with a fluid-transfer article configured to provide a flavoured vapour and/or aerosol upon heating of the fluid-transfer article by the heater 24 of the apparatus 12. A precursor material for forming the flavoured vapour and/or aerosol upon heating is held within pores, spaces, channels and/or conduits within the fluid-transfer article. The precursor material may be extracted from a tobacco plant starting material using a supercritical fluid extraction process. Optionally, the precursor material is nicotine-free and comprises tobacco-flavours extracted from the tobacco plant starting material. Further optionally, the extracted nicotine-free precursor material (e.g. flavours only) could have nicotine added thereto prior to loading of the precursor material into the substrate of the carrier unit. Further optionally, flavours and physiologically active material may be extracted from plants other than tobacco plants.

## Claims

1. An aerosol-generation apparatus (12) comprising a heater (24) and a fluid-transfer article (34), the fluid-transfer article (34) having a first region (34a) for holding an aerosol precursor and for transferring said aerosol precursor to an activation surface (38) of a second region (34b) of said article (34), said activation surface (38) configured for thermal interaction with a heating surface of said heater (24); **characterised in that** said heating surface includes at least one discontinuity therein forming a corresponding at least one channel (40), the or each said channel being configured for providing a fluid-flow pathway across said activation surface, said heating surface being configured such that, when the fluid transfer article (34) is arranged with respect to said heating surface for thermal interaction therebetween, the or each said channel (40) opposes said activation surface (38) and opens towards said activation surface (38).

2. An aerosol-generation apparatus (12) according to claim 1, wherein said heating surface is configured such that the or each said discontinuity is spaced apart from said activation surface (38).

3. An aerosol-generation apparatus (12) according to claim 1 or claim 2, wherein the or each said channel (40) is at least partly defined by a pair of spaced apart side walls, and an arcuate surface portion extending between said wall portions to form a ceiling portion of said channel (40).

4. An aerosol-generation apparatus (12) according to claim 3, wherein said arcuate surface portion blends smoothly with each of said side walls, thereby eliminating a sharp corner therebetween.

5. An aerosol-generation apparatus (12) according to claim 1 or claim 2, wherein the or each channel (40) is at least partially defined by a pair of spaced apart side walls and a flat surface portion, said flat surface portion extending between said wall portions to form a ceiling portion of said channel (40).

6. An aerosol-generation apparatus (12) according to claim 1 or claim 2, wherein the or each channel (40) is at least partially defined by a pair of side walls, said side walls being inclined relative to each other to meet at an apex portion of said channel (40).

7. An aerosol-generation apparatus (12) according to any one of claims 3 to 6, wherein said side walls are substantially planar.

8. An aerosol-generation apparatus (12) according to any preceding claim, wherein at least said second region of said fluid-transfer article (34) is formed from a polymeric wicking material.

9. An aerosol-generation apparatus (12) according to claim 8, wherein said first and second regions (34a, 34b) of said fluid-transfer article (34) are both formed from said polymeric wicking material.

10. An aerosol-generation apparatus (12) according to claim 8, wherein said polymeric wicking material is porous.

11. An aerosol-generation apparatus (12) according to any one of claims 8 to 10, wherein said polymeric wicking material is configured such that the pore diameter in said first region (34a) is greater than the pore diameter in said second region (34b).

12. An aerosol-generation apparatus (12) according to any one of claims 8 to 11, wherein said polymeric wicking material is heat resistant.

13. An aerosol-generation apparatus (12) according to any one of claims 8 to 12, wherein said polymeric wicking material is a hydrophilic material that is configured to transfer fluid from said first region (34a) to said second region (34b).

14. An aerosol-generation apparatus (12) according to any one of claims 8 to 13, wherein said polymeric wicking material is of greater hydrophilicity in said second region (34b) than said first region (34a).

15. An aerosol delivery system (10) comprising an aerosol-generation apparatus (12) according to any of the preceding claims and a carrier (14), the carrier (14) having a housing (12) containing said heater (24) and said fluid-transfer article (34).

16. An aerosol delivery system (10) according to claim 15, wherein said housing (32) has an inlet (50) and an outlet, and said air-flow pathway extends to said inlet (50) and said outlet.

## Patentansprüche

1. Aerosolerzeugungsvorrichtung (12), umfassend ein Heizelement (24) und einen Fluidtransportartikel (34), wobei der Fluidtransportartikel (34) eine erste Region (34a) zum Halten eines Aerosolvorläufers und zum Transportieren des Aerosolvorläufers zu einer Aktivierungsfläche (38) einer zweiten Region (34b) des Artikels (34) aufweist, wobei die Aktivierungsfläche (38) zur thermischen Wechselwirkung mit einer Heizfläche des Heizelements (24) ausgelegt ist; **dadurch gekennzeichnet, dass** die Heizfläche zumindest eine Diskontinuität in dieser umfasst, die zumindest einen entsprechenden Kanal (40) ausbildet, wobei der oder jeder Kanal zum Bereitstellen eines Fluidströmungswegs über die Aktivierungsfläche ausgelegt ist, wobei die Heizfläche derart ausgelegt ist, dass, wenn der Fluidtransportartikel (34) in Bezug auf die Heizfläche zur thermischen Wechselwirkung zwischen diesen angeordnet ist, der oder jeder Kanal (40) der Aktivierungsfläche (38) gegenüberliegt und sich in Richtung der Aktivierungsfläche (38) öffnet.

2. Aerosolerzeugungsvorrichtung (12) nach Anspruch 1, wobei die Heizfläche derart ausgelegt ist, dass die oder jede Diskontinuität von der Aktivierungsfläche (38) beabstandet ist.

3. Aerosolerzeugungsvorrichtung (12) nach Anspruch 1 oder 2, wobei der oder jeder Kanal (40) zumindest teilweise durch ein Paar von voneinander beabstandeten Seitenwänden definiert ist und ein bogenförmiger Oberflächenabschnitt sich zwischen den Wandabschnitten erstreckt, um einen Deckenabschnitt des Kanals (40) auszubilden.

4. Aerosolerzeugungsvorrichtung (12) nach Anspruch 3, wobei der bogenförmige Oberflächenabschnitt sich nahtlos in jede der Seitenwände einfügt, wodurch eine scharfe Ecke zwischen diesen beseitigt wird.

5. Aerosolerzeugungsvorrichtung (12) nach Anspruch 1 oder 2, wobei der oder jeder Kanal (40) zumindest teilweise durch ein Paar von voneinander beabstandeten Seitenwänden und einen flachen Oberflächenabschnitt definiert ist, wobei der flache Oberflächenabschnitt sich zwischen den Wandabschnitten erstreckt, um einen Deckenabschnitt des Kanals (40) auszubillden.

6. Aerosolerzeugungsvorrichtung (12) nach Anspruch 1 oder 2, wobei der oder jeder Kanal (40) zumindest teilweise durch ein Paar von Seitenwänden definiert ist, wobei die Seitenwände relativ zueinander geneigt sind, um in einem Scheitelpunktabschnitt des Kanals (40) aufeinanderzutreffen.

7. Aerosolerzeugungsvorrichtung (12) nach einem der Ansprüche 3 bis 6, wobei die Seitenwände im Wesentlichen planar sind.

8. Aerosolerzeugungsvorrichtung (12) nach einem der vorangegangenen Ansprüche, wobei die zumindest zweite Region des Fluidtransportartikels (34) aus einem Polymerdochtmaterial ausgebildet ist.

9. Aerosolerzeugungsvorrichtung (12) nach Anspruch 8, wobei die erste und zweite Region (34a, 34b) des Fluidtransportartikels (34) beide aus dem Polymerdochtmaterial ausgebildet sind.

10. Aerosolerzeugungsvorrichtung (12) nach Anspruch 8, wobei das Polymerdochtmaterial porös ist.

11. Aerosolerzeugungsvorrichtung (12) nach einem der Ansprüche 8 bis 10, wobei das Polymerdochtmaterial derart ausgelegt ist, dass der Porendurchmesser in der ersten Region (34a) größer ist als der Porendurchmesser in der zweiten Region (34b).

12. Aerosolerzeugungsvorrichtung (12) nach einem der Ansprüche 8 bis 11, wobei das Polymerdochtmaterial hitzebeständig ist.

13. Aerosolerzeugungsvorrichtung (12) nach einem der Ansprüche 8 bis 12, wobei das Polymerdochtmaterial ein hydrophiles Material ist, das dazu ausgelegt ist, Fluid von der ersten Region (34a) zur zweiten Region (34b) zu transportieren.

14. Aerosolerzeugungsvorrichtung (12) nach einem der Ansprüche 8 bis 13, wobei das Polymerdochtmaterial in der zweiten Region (34b) eine größere Hydrophilie als in der ersten Region (34a) aufweist.

15. Aerosolabgabesystem (10), umfassend eine Aerosolerzeugungsvorrichtung (12) nach einem der vorangegangenen Ansprüche und einen Träger (14), wobei der Träger (14) ein Gehäuse (12) aufweist, welches das Heizelement (24) und den Fluidtransportartikel (34) enthält.

16. Aerosolabgabesystem (10) nach Anspruch 15, wobei das Gehäuse (32) einen Einlass (50) und einen Auslass aufweist und sich der Luftströmungsweg zu dem Einlass (50) und dem Auslass erstreckt.

## Revendications

1. Appareil de génération d'aérosol (12) comprenant un dispositif de chauffage (24) et un article de transfert de fluide (34), l'article de transfert de fluide (34) présentant une première région (34a) destinée à contenir un précurseur d'aérosol et à transférer ledit précurseur d'aérosol vers une surface d'activation (38) d'une seconde région (34b) dudit article (34), ladite surface d'activation (38) étant configurée pour une interaction thermique avec une surface chauffante dudit dispositif de chauffage (24) ; **caractérisé en ce que** ladite surface chauffante inclut au moins une discontinuité formant en son sein au moins un canal correspondant (40), le ou chaque canal étant configuré pour fournir un trajet d'écoulement de fluide à travers ladite surface d'activation, ladite surface chauffante étant configurée de telle sorte que lorsque l'article de transfert de fluide (34) est agencé par rapport à ladite surface chauffante pour une interaction thermique entre ceux-ci, le ou chaque canal (40) s'oppose à ladite surface d'activation (38) et s'ouvre vers ladite surface d'activation (38).

2. Appareil de génération d'aérosol (12) selon la revendication 1, dans lequel ladite surface chauffante est configurée de telle sorte que la ou chaque discontinuité est espacée de ladite surface d'activation (38).

3. Appareil de génération d'aérosol (12) selon la revendication 1 ou la revendication 2, dans lequel le ou chaque canal (40) est au moins partiellement défini par une paire de parois latérales espacées, et une partie de surface arquée s'étendant entre lesdites parties de paroi pour former une partie de plafond dudit canal (40).

4. Appareil de génération d'aérosol (12) selon la revendication 3, dans lequel ladite partie de surface arquée se fond en douceur avec chacune desdites parois latérales, en éliminant ainsi un coin aigu entre celles-ci.

5. Appareil de génération d'aérosol (12) selon la revendication 1 ou la revendication 2, dans lequel le ou chaque canal (40) est au moins partiellement défini par une paire de parois latérales espacées et une partie de surface plate, ladite partie de surface plate s'étendant entre lesdites parties de paroi pour former une partie de plafond dudit canal (40).

6. Appareil de génération d'aérosol (12) selon la revendication 1 ou la revendication 2, dans lequel le ou chaque canal (40) est défini au moins partiellement par une paire de parois latérales, lesdites parois latérales étant inclinées l'une par rapport à l'autre pour se rencontrer au niveau d'une partie de sommet dudit canal (40).

7. Appareil de génération d'aérosol (12) selon l'une quelconque des revendications 3 à 6, dans lequel lesdites parois latérales sont sensiblement planes.

8. Appareil de génération d'aérosol (12) selon l'une quelconque des revendications précédentes, dans lequel au moins ladite seconde région dudit article de transfert de fluide (34) est formée à partir d'un matériau à effet de mèche polymère.

9. Appareil de génération d'aérosol (12) selon la revendication 8, dans lequel lesdites première et seconde régions (34a, 34b) dudit article de transfert de fluide (34) sont toutes deux formées à partir dudit matériau à effet de mèche polymère.

10. Appareil de génération d'aérosol (12) selon la revendication 8, dans lequel ledit matériau à effet de mèche polymère est poreux.

11. Appareil de génération d'aérosol (12) selon l'une quelconque des revendications 8 à 10, dans lequel ledit matériau à effet de mèche polymère est configuré de telle sorte que le diamètre de pore dans ladite première région (34a) est supérieur au diamètre de pore dans ladite seconde région (34b).

12. Appareil de génération d'aérosol (12) selon l'une quelconque des revendications 8 à 11, dans lequel ledit matériau à effet de mèche polymère est thermo-résistant.

13. Appareil de génération d'aérosol (12) selon l'une quelconque des revendications 8 à 12, dans lequel ledit matériau à effet de mèche polymère est un matériau hydrophile qui est configuré pour transférer un fluide de ladite première région (34a) à ladite seconde région (34b).

14. Appareil de génération d'aérosol (12) selon l'une quelconque des revendications 8 à 13, dans lequel ledit matériau à effet de mèche polymère est d'une plus grande hydrophilie dans ladite seconde région (34b) que dans ladite première région (34a).

15. Système de distribution d'aérosol (10) comprenant un appareil de génération d'aérosol (12) selon l'une quelconque des revendications précédentes et un support (14), le support (14) présentant un logement (12) contenant ledit dispositif de chauffage (24) et ledit article de transfert de fluide (34).

16. Système de distribution d'aérosol (10) selon la revendication 15, dans lequel ledit logement supplémentaire (32) présente une entrée (50) et une sortie, et ledit trajet d'écoulement d'air s'étend vers ladite entrée (50) et ladite sortie.
